# EUROPÄISCHE PATENTANMELDUNG

(11) **EP 3 409 655 A1**
(43) Veröffentlichungstag der Anmeldung: **05.12.2018**
(21) Anmeldenummer: 17173746.3
(22) Anmeldetag: 31.05.2017
(51) Int. Cl.: C07C 49/08, C07C 45/82, B01D 3/14

(54) **VERFAHREN UND VORRICHTUNG ZUR AUFREINIGUNG VON ACETON/WASSERGEMISCHEN MITTELS DOUBLE EFFEKT DESTILLATION**

(71) Anmelder: Rhodia Acetow GmbH, 79108 Freiburg (DE)
(72) Erfinder: HUMMEL, Andreas, 79108 Freiburg (DE); KRUMREY, Thomas, 79331 Teningen (DE)
(74) Vertreter: Trinks, Ole

(57) **Zusammenfassung**

Die vorliegende Erfindung betrifft ein Verfahren zur Abtrennung von Aceton aus Aceton/Wassergemischen, bei dem ein Teilstrom des Aceton/Wassergemisches in einer bei Überdruck betriebenen Kolonne aufkonzentriert wird, um ein Produkt mit einer Acetonkonzentration von mindestens 80 Gew.-% zu erhalten, und wobei ein Teilstrom des Aceton/Wassergemisches in einer Kolonne, die bei Normaldruck betrieben wird, aufkonzentriert wird. Das Kopfprodukt aus der Kolonne, die bei Überdruck betrieben wird, wird dabei über einen Wärmetauscher zum Erwärmen des Sumpfprodukts der Kolonne, die unter Normaldruck betrieben wird, genutzt; anschließend wird das Kopfprodukt oberhalb der Zufuhr des Teilstroms des Aceton/Wassergemisches in die Kolonne, die unter Normaldruck betrieben wird, eingeleitet. Durch diese Verfahrensführung lässt sich der spezifische Energiebedarf für die Acetontrennung signifikant reduzieren, was mit erheblichen Kostenvorteilen verbunden ist. Die vorliegende Erfindung betrifft ebenfalls eine Vorrichtung zur Durchführung eines entsprechenden Verfahrens.

## Beschreibung

Die vorliegende Erfindung betrifft ein Verfahren zur Abtrennung von Aceton aus Aceton/Wassergemischen unter Nutzung der DED-Technik (Double Effect Destillation), bei der zwei Rektifikationskolonnen bei unterschiedlichem Druck gefahren werden, so dass die Abwärme des Kopfprodukts einer unter Druck gefahrenen Kolonne zur Erwärmung des Sumpfprodukts einer bei niedrigerem Druck gefahrenen Kolonne genutzt werden kann. Die vorliegende Erfindung betrifft weiterhin eine Vorrichtung zur Durchführung eines entsprechenden Verfahrens.

Zum Zweck der Energieeinsparung bei Destillationsanlagen ist die sogenannte DED-Technik (Double Effect Distillation) seit Langem bekannt. Bei dieser Technik werden zwei (oder mehrere) Anlagen bei verschiedenen Drücken gefahren, wo-durch sich verschiedene Siedetemperaturen im Kopf und Sumpf der jeweiligen Kolonnen ergeben. Durch geeignete Abstimmung der Drücke kann eine Wärmekopplung zwischen diesen Kolonnen aufgebaut werden, so dass das Kopfprodukt einer bei höherem Druck betriebenen Kolonne den Sumpf einer bei niedrigerem Druck gefahrenen Kolonne beheizen kann. Durch diese Maßnahme lässt sich entsprechend Heizenergie einsparen.

Bei der Acetonrückgewinnung stellt sich hierbei allerdings das Problem, dass die Siedetemperaturen von Kopf und Sumpfprodukt insbesondere bei Aceton/Wassergemischen relativ weit auseinander liegen (55°C zu 102°C bei Normaldruck). Dies hat zur Folge, dass eine "Vakuumkolonne" in einer DED-Anlage so weit im Vakuum betrieben werden muss, dass die Sumpftemperatur mindestens 5 Kelvin unter der Kopftemperatur von 55°C einer Normaldruckkolonne liegt. Dies wird bei etwa 80 mbar absolut erreicht, ist aber mit dem erheblichen Nachteil verbunden, dass aufgrund des niedrigen Vakuums die erforderlichen Kolonnen sehr groß dimensioniert werden müssen, was mit hohen Investitionskosten verbunden ist. Zudem erfolgt die Kondensation am Kopf bei diesen Vakuum-Kolonnen bei etwa 0°C, so dass die Verwendung von preisgünstigen Kühlmedien, wie z.B. Kühlturmwasser bzw. Luftkühlung, nicht mehr möglich ist. Eine Wärmerückgewinnung mit dem Zulauf ist ebenfalls nicht mehr möglich.

Eine alternative Variante einer Double Effect Distillation besteht in der Kopplung einer Normaldruckkolonne mit einer Druckkolonne. Hierbei muss der Druck auf mindestens 5 bar absolut angehoben werden, damit eine Wärmekopplung, wie oben beschrieben, möglich wird. Bei diesen Drücken bildet sich jedoch ein Azeotrop von Wasser und Aceton, so dass die Reinheit des über die Druckkolonne erhaltenen Acetons signifikant geringer ist als die Reinheit des über die Normaldruckkolonne erhaltenen Acetons. Darüber hinaus steigt bei höheren Temperaturen das Risiko der Bildung von Abbauprodukten des Acetons, wie insbesondere von Diacetonalkohol und Mesityloxid.

Bisher wurde eine solche Verfahrensführung z.B. in der FR 2549043 für die Aufreinigung von wässrigen Lösungen von Butanol und Aceton vorgeschlagen, wobei in einer ersten Stufe die organischen Bestandteile Butanol und Aceton auf einen Gehalt von etwa 60% angereichert werden, während in einer zweiten Stufe Ace-ton und Butanol vollständig voneinander getrennt werden. Die FR 2549043 gibt an, dass die erste Destillationsstufe mit zwei Destillationssäulen ausgestaltet sein kann, wobei eine erste Destillationssäule bei erhöhtem Druck von 3 bis 10 bar absolut und eine zweite Destillationssäule bei einem Druck von 0,5 bis 1,5 bar absolut gefahren wird. Da dieses Verfahren in der ersten Stufe jedoch kein hin-länglich reines Aceton als Endprodukt liefert (der Wassergehalt nach der ersten Stufe beträgt etwa 40%; reines Aceton wird erst in der zweiten Destillationsstufe generiert), ist das Verfahren der FR 2549043 mit dem Nachteil verbunden, dass eine weitere Destillationsstufe erforderlich ist, um die erforderliche Reinheit zu erreichen.

Eine Kombination beider Alternativen könnte einen Kompromiss darstellen, wobei eine Kolonne mit einem leichten Vakuum mit einer Druckkolonne, die bei leichtem Überdruck gefahren wird, kombiniert wird.

Bei bestehenden Installationen mit einer bereits vorhandenen Normaldruckkolonne ist diese Kombination jedoch mit erheblich höheren Investitionskosten verbunden als die Kombination einer Normaldruckkolonne mit einer Druckkolonne bzw. einer Niederdruckkolonne und einer Normaldruckkolonne.

Vor dem Hintergrund dieses Standes der Technik bestand ein Bedarf für die Bereitstellung eines energieökonomischen Verfahrens zur Aufreinigung und Abtrennung von Aceton aus Aceton/Wassergemischen, bei dem die vorstehend geschilderten Nachteile weitestgehend vermieden werden und ein möglichst geringer spezifischer Energiebedarf realisiert werden kann. Die vorstehende Erfindung befasst sich mit diesem Bedarf.

Gemäß einem ersten Aspekt betrifft die vorstehende Erfindung demzufolge ein Verfahren zur Abtrennung von Aceton aus Aceton/Wassergemischen, umfassend
- Aufkonzentrieren eines Teilstroms des Aceton/Wassergemisches in mindestens einer Kolonne, die bei Überdruck betrieben wird, wobei ein Produkt mit einer Acetonkonzentration von mindestens 80 Gew.-% erhalten wird,
- Aufkonzentrieren eines Teilstroms des Aceton/Wassergemisches in mindestens einer Kolonne, die bei Normaldruck betrieben wird,
wobei das Kopfprodukt aus der Kolonne, die bei Überdruck betrieben wird, über einen Wärmetauscher zum Erwärmen des Sumpfprodukts der Kolonne, die unter Normaldruck betrieben wird, genutzt wird, und wobei das Kopfprodukt anschließend oberhalb der Zufuhr des Teilstroms des Aceton/Wassergemisches in die Kolonne, die unter Normaldruck betrieben wird, eingeleitet wird.

Demzufolge entspricht das erfindungsgemäße Verfahren im Wesentlichen einem regulären Destillationsverfahren unter Nutzung der Double Effect-Destillations-technik, bei dem in Kauf genommen wird, dass sich im Rahmen der Aufkonzentration eines Teilstroms des Aceton/Wassergemisches in einer Kolonne, die bei Überdruck betrieben wird, keine optimale Trennung von Aceton und Wasser realisieren lässt. Dieser Nachteil wird jedoch dadurch kompensiert, dass das zumindest in Bezug auf Aceton wesentlich angereicherte Aceton/Wassergemisch in die Aufkonzentrierungskolonne, die bei Normaldruck betrieben wird, eingeleitet wird, so dass dort eine vollständige Trennung ermöglicht wird. Diese Prozessführung hat insbesondere bei in Bezug auf Aceton niedrig konzentrierten Aceton/Wassergemischen den Vorteil, dass nur eine geringe Menge eines schon relativ stark aufkonzentrierten Acetons als Kopfprodukt der unter Druck betriebenen Kolonne in die unter Normaldruck betriebene Kolonne eingeleitet wird. Über den Wärmetauscher wird die in der Druckkolonne eingesetzte Energie weitestgehend oder im Wesentlichen vollständig wiederverwertet. Die Zuführung des in Bezug auf Aceton angereicherten Aceton/Wassergemisches bewirkt zudem, dass das Rücklaufverhältnis in der bei Normaldruck betriebenen Kolonne stark reduziert werden kann, wo-durch sich der Gesamtenergiebedarf der kombinierten Anlage weiter reduziert. Von Vorteil ist dabei auch, dass sich die hydraulische Belastung des Kolonnenteils, in den das Acetongemisch zugeführt wird nur unwesentlich ändert.

Wenn im Vorstehenden von "oberhalb" oder "unterhalb" in Bezug auf Kolonnen die Rede ist, so bedeutet "oberhalb" in diesem Zusammenhang, dass "oben" in Bezug auf die Kolonne den Teil der Kolonne, der bei niedrigerer Temperatur betrieben wird, und "unten" den Teil der Kolonne, der bei höherer Temperatur betrieben wird, bezeichnet. Demzufolge wird das Sumpfprodukt im unteren Bereich der Kolonne und das Kopfprodukt im oberen Bereich der Kolonne erhalten.

Unter "Normaldruck" ist im Kontext der vorliegenden Erfindung ein Druck im Bereich von 0,5 bis 1,5 und bevorzugt 0,8 bis 1,2 bar absolut zu verstehen. Die Kolonne, die bei Überdruck betrieben wird, wird im Rahmen der vorliegenden Erfindung in der Regel bei einem Druck im Bereich von 4 bis 11 bar absolut betrieben, wobei jedoch ein Überdruck im Bereich von 4,5 bis 8 bar absolut und insbesondere von 5 bis 7 bar absolut als bevorzugt angegeben werden kann. Als am meisten bevorzugt gilt im Rahmen der vorliegenden Erfindung für die bei Überdruck betriebene Kolonne ein Druck von etwa 6 bar absolut.

Unter "Aceton/Wassergemisch" ist im Kontext der vorliegenden Erfindung ein Gemisch, welches aus Aceton und Wasser besteht, zu verstehen; desweiteren ist darunter ebenfalls ein Gemisch zu verstehen, das im Wesentlichen aus Aceton und Wasser besteht. "Im Wesentlichen" bedeutet hier ein Gemisch, das zu mindestens 95% Gew.% aus Aceton und Wasser, bevorzugt zu 97 bis 99,9 Gew.-% aus Wasser und Aceton besteht. Weitere Inhaltsstoffe, die in dem Wasser/Acetongemisch mit bis zu 5 Gew.% enthalten sein können, sind, z.B. Kohlenwasserstoffe, beispielsweise sog. Spinnöl, das bei der Herstellung von Celluloseacetatfasern eingesetzt wird, Diacetonalkohol oder Mesityloxid. Das Aceton/Wassergemisch, aus welchem in dem Verfahren der vorliegenden Erfindung Aceton abgetrennt wird, stammt bevorzugt aus einem Spinnverfahren zur Herstellung von Celluloseacetatfasern, wie es beispielsweise in P. Rustemeyer, Macromol. Symp. 2004, 208, 267-291 beschrieben wird.

Im Rahmen der vorliegenden Erfindung ist es weiterhin zweckmäßig, wenn in der Kolonne, die unter Überdruck betrieben wird, ein Kopfprodukt erhalten wird, das eine Acetonkonzentration von mindestens 90 Gew.-%, bevorzugt 90 bis 99,9 Gew.-%, stärker bevorzugt 93 bis 98 Gew.-% und weiter bevorzugt 94 Gew.-% bis 96 Gew.-%, aufweist.

Im Rahmen des geschilderten Verfahrens ist es zweckmäßig, wenn die Abwärme aus dem Sumpfprodukt der bei Überdruck betriebenen Kolonne für das Vorerwärmen von in den Prozess zugeführtem Aceton/Wassergemisch genutzt wird. Besonders zweckmäßig ist es, wenn das aus der Kolonne, die bei Überdruck betrieben wird, erhaltene Sumpfprodukt zur Vorerwärmung des in die Kolonne eingebrachten Aceton/Wassergemisches genutzt wird. Eine entsprechende Nutzung der Abwärme lässt sich mit Hilfe eines Wärmetauschers, in dem die Abwärme aus dem Sumpfprodukt an das der Kolonne zugeführte Aceton/Wassergemisch abgegeben werden kann, realisieren.

Im Rahmen des vorstehend geschilderten Verfahrens sind die bei Überdruck und Normaldruck betriebenen Kolonnen zweckmäßig so auszugestalten, dass eine ausreichende Trennung von Aceton und Wasser gewährleistet ist.

Für die Kolonne, die bei Überdruck betrieben wird, ist es hierbei bevorzugt, wenn sie 30 bis 60 und bevorzugt 40 bis 50 theoretische Böden aufweist.

Für die Kolonne, die bei Normaldruck betrieben wird, gilt eine Anzahl von 20 bis 40 und bevorzugt 25 bis 35 theoretischen Böden als geeignet.

Die Temperatur im Sumpfprodukt der Kolonne, die bei Überdruck betrieben wird, beträgt im Rahmen des hier angegebenen Verfahrens vorzugsweise 130 bis 180°C, insbesondere 150 bis 170°C und meist bevorzugt etwa 160°C. Die Temperatur des Kopfprodukts dieser Kolonne beträgt zweckmäßig etwa 100 bis 130°C und insbesondere etwa 105 bis 120°C. Um eine ausreichend effiziente Wärmeübertragung an das Sumpfprodukt der Kolonne, die bei Normaldruck betrieben wird, zu gewährleisten, sollte die Temperatur des Kopfprodukts zudem bevorzugt mindestens 5°C und insbesondere mindestens 10°C höher sein als die Temperatur des Sumpfprodukts in der Kolonne, die bei Normaldruck betrieben wird.

Das Verfahren ist erfindungsgemäß zweckmäßig so auszugestalten, dass das Ace-ton in der mindestens einen Kolonne, die bei Normaldruck betrieben wird, auf eine Konzentration von mindestens 98 Gew.-% und besonders bevorzugt mindestens 98,5 Gew.-%, stärker bevorzugt mindestens 98,5 bis 99,9 Gew.-% aufkonzentriert wird. Alternativ oder zusätzlich dazu ist es zweckmäßig, wenn die Kolonne, die bei Normaldruck betrieben wird, mit einem Rücklaufverhältnis im Bereich von 2 bis 5 und bevorzugt von 3 bis 4 betrieben wird.

Das im Rahmen des erfindungsgemäßen Verfahrens aus der Kolonne, die bei Normaldruck betrieben wird, erhaltene Kopfprodukt wird zweckmäßig in einem Kühler kondensiert, um flüssiges Aceton zu erhalten. Hierzu wird bevorzugt reguläres Kühlwasser verwendet.

Durch die Auslegung des erfindungsgemäßen Verfahrens, bei der eine Wärmekopplung der mindestens einen Kolonne, die bei Überdruck betrieben wird und der mindestens einen Kolonne, die bei Normaldruck betrieben wird, erfolgt, wird eine erhebliche Energieeinsparung möglich.

Der spezifische Energieverbrauch des erfindungsgemäßen Verfahrens lässt sich noch weiter senken, wenn eine dritte Kolonne vorgesehen wird, die unter Vakuum betrieben wird. In dieser Ausführungsform der Erfindung ist das erfindungsgemäße Verfahren daher so ausgestaltet, dass es neben den oben genannten Schritten des Weiteren umfasst:
- Aufkonzentrieren eines Teilstroms des Aceton/Wassergemisches in mindestens einer Kolonne, die unter Vakuum betrieben wird,
wobei das Kopfprodukt aus der Kolonne, die bei Normaldruck betrieben wird, über einen Wärmetauscher zum Erwärmen des Sumpfproduktes der Kolonne, die unter Vakuum betrieben wird, genutzt wird.

Unter "Vakuum" ist in Kontext der vorliegenden Erfindung ein Druck im Bereich von <0,5, insbesondere 0,05 bis <0,5 bar absolut zu verstehen.

Diese Ausführungsform der Erfindung führt durch das Vorsehen einer dritten Kolonne zu einer Erhöhung der Investitionskosten, senkt jedoch im Gegenzug die Energiekosten für den laufenden Betrieb noch weiter.

Zusätzlich kann in dieser Ausführungsform der Erfindung, bei der mindestens drei Kolonnen vorgesehen sind, das Verfahren so ausgelegt werden, dass das Sumpfprodukt aus der Kolonne, die bei Normaldruck betrieben wird, unterhalb der Zufuhr des Teilstroms des Aceton/Wassergemisches in die Kolonne, die unter Vakuum betrieben wird, eingeleitet werden kann.

Diese Ausführungsform hat den zusätzlichen Vorteil, dass eine noch stärkere Abreicherung von Aceton im Sumpfprodukt der Kolonne, die bei Normaldruck betrieben wird, erreicht wird. Dies ist unter umweltschutztechnischen Gesichtspunkten und damit verbunden auch unter wirtschaftlichen Gesichtspunkten vorteilhaft.

Ein weiterer Aspekt der vorliegenden Erfindung betrifft eine Vorrichtung zur Durchführung eines Verfahrens, wie es vorstehend beschrieben wurde, wobei die Vorrichtung Folgendes umfasst:
- mindestens eine Kolonne 1 zur Auftrennung eines Teilstroms eines Aceton/ Wassergemisches, die für einen Betrieb mit Überdruck ausgelegt ist,
- mindestens eine Kolonne 2 zur Auftrennung eines Teilstroms eines Aceton/ Wassergemisches, die für einen Betrieb mit Normaldruck ausgelegt ist,
- Zuleitungen für Teilströme 7, 8 eines Aceton/Wassergemisches zu den Trennkolonnen,
- mindestens einen Wärmetauscher 3, der in fließender Verbindung mit dem Kopf der Kolonne 1, die für einen Betrieb mit Überdruck ausgelegt ist, und mit dem Boden der Kolonne 2, die für einen Betrieb mit Normaldruck ausgelegt ist, steht, sodass ein Wärmeaustausch des Kopfprodukts der Kolonne 1, die für einen Betrieb mit Überdruck ausgelegt ist, und des Sumpfprodukts der Kolonne 2, die für einen Betrieb mit Normaldruck ausgelegt ist, ermöglicht wird,
- eine Zuleitung 5 für das Kopfprodukt der Kolonne 1, die für einen Betrieb mit Überdruck ausgelegt ist, mit der der Wärmetauscher 3 mit dem oberen Teil der Kolonne 2, die für einen Betrieb mit Normaldruck ausgelegt ist, verbunden ist.

Die Zuleitung 5 ist zweckmäßig oberhalb der Zuleitung 7 von einem Teilstrom eines Aceton/Wassergemisches zur Kolonne 2, die für einen Betrieb mit Normal-druck ausgelegt ist, an der Kolonne 2 angebracht.

In einer Ausführungsform ist das Verfahren so ausgestaltet, dass im Bereich der Zuleitung 5 eine Auffangvorrichtung für das Kopfprodukt vorgesehen ist, in der dieses vor der Zuleitung in die Kolonne 2 auf den dort vorhandenen Druck entspannt wird. Die Auffangvorrichtung, z.B. in Form eines Kessel oder äquivalenten Reservoirs, ist in dieser Ausführungsform im Bereich der Zuleitung 5 zwischen dem Wärmetauscher 3 und der Kolonne 2 positioniert.

Darüber hinaus ist es für die geschilderte Vorrichtung bevorzugt, wenn sie eine Kondensationsvorrichtung für in der Kolonne, die für einen Betrieb mit Normal-druck ausgelegt ist, generiertes Kopfprodukt aufweist.

Schließlich ist es für die erfindungsgemäße Vorrichtung bevorzugt, dass sie einen Wärmetauscher 10 aufweist, der in fließender Verbindung mit dem Sumpfprodukt der Kolonne 1, die für einen Betrieb mit Überdruck ausgelegt ist, und mit der Zuleitung 8 für den Teilstrom eines Aceton/Wassergemisches zu dieser Kolonne steht.

Im Folgenden soll die vorliegende Erfindung unter Verweis auf die Figuren näher erläutert werden.

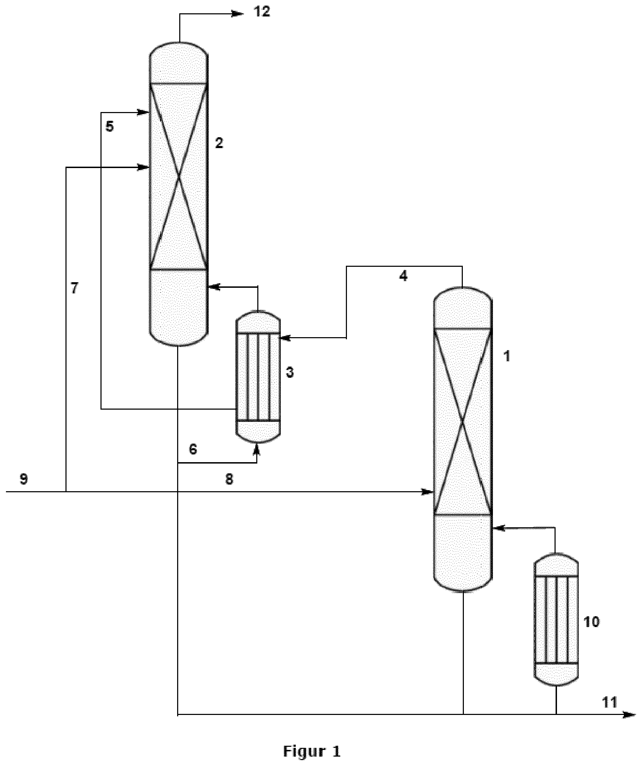

Figur 1 zeigt eine erfindungsgemäße Vorrichtung, bei der eine Kolonne 1, die für einen Betrieb mit Überdruck ausgelegt ist, und eine Kolonne 2, die für einen Betrieb mit Normaldruck ausgelegt ist, miteinander verschaltet sind. Das Kopfprodukt, das vorzugsweise eine Acetonkonzentration von mindestens 90 Gew.-%, bevorzugt 90 bis 99,9 Gew.-%, stärker bevorzugt 93 Gew.-% bis 98 Gew.-%, und weiter bevorzugt 94 Gew.-% bis 96 Gew.-% aufweist, wird über die Ableitung 4 in einen Wärmetauscher 3 überführt und von dort über die Zuleitung 5 in die Kolonne 2 eingeleitet. Aus einer gemeinsamen Zuleitung 9 wird über die Teilleitungen 7 und 8 Aceton/Wassergemisch in die Kolonnen 1 und 2 geleitet. Der Wärmetauscher 3 ist ebenfalls über den Kreislauf 6 mit dem Sumpfprodukt der Kolonne 2 verbunden, um über die Abgabe aus Abwärme aus dem Kopfprodukt der Kolonne 1 an das Sumpfprodukt der Kolonne 2 einen Wärmeaustausch zu ermöglichen. Aus der Kolonne 2 wird als Kopfprodukt im Wesentlichen reines Aceton über die Ableitung 12 erhalten. Unter im Wesentlichen reinem Aceton wird im Sinne der vorliegenden Anmeldung vorzugsweise ein Produkt verstanden, das einen Acetongehalt von mindestens 98 Gew.-%, vorzugsweise mindestens 98,5 Gew.-%, insbesondere 98,5 bis 99,9 Gew.-% aufweist. Das in der Trennkolonne 1 generierte Sumpfprodukt ist ebenfalls in einem Kreislauf mit einem Wärmetauscher 10 eingebunden, indem dem Sumpfprodukt zusätzliche Wärme beispielsweise über entsprechend hoch erhitzten Dampf zugeführt werden kann. Überschüssiges Sumpfprodukt wird als Abwasser über die Ableitung 11 aus der erfindungsgemäßen Vorrichtung freigesetzt. Die Abwasserleitung 11 ist zudem mit dem Sumpfproduktkreislauf 6 der Kolonne 2 verbunden, um dort anfallendes überschüssiges Sumpfprodukt abzuführen.

Nach angestellten Berechnungen kann durch die Verfahrensführung und Vorrichtung gemäß der vorliegenden Erfindung eine signifikante Reduktion des spezifischen Energiebedarfs um etwa 35 bis 45% erzielt werden. Gegenüber den weiter oben beschriebenen theoretischen Alternativen verringert sich das Investitionsvolumen ebenfalls erheblich, da nur eine neue Kolonne benötigt wird und ggf. vorhandene Normaldruckkolonnen ohne größere Änderung eingebunden werden können.

In einer Ausführungsform der erfindungsgemäßen Vorrichtung ist eine dritte Kolonne vorgesehen, die für den Betrieb unter Vakuum ausgelegt wird. In dieser Ausführungsform umfasst die erfindungsgemäße Vorrichtung folgendes:
- mindestens eine Kolonne 1 zur Auftrennung eines Teilstroms eines Aceton/ Wassergemisches, die für einen Betrieb mit Überdruck ausgelegt ist,
- mindestens eine Kolonne 2a zur Auftrennung eines Teilstroms eines Aceton/Wassergemisches, die für einen Betrieb mit Normaldruck ausgelegt ist,
- mindestens eine Kolonne 2b zur Auftrennung eines Teilstroms eines Aceton/Wassergemisches, die für einen Betrieb unter Vakuum ausgelegt ist,
- Zuleitungen für Teilströme 7a, 7b, 8 eines Aceton/Wassergemisches zu den Trennkolonnen,
- mindestens einen Wärmetauscher 3a, der in fließender Verbindung mit dem Kopf der Kolonne 1, die für einen Betrieb mit Überdruck ausgelegt ist, und mit dem Boden der Kolonne 2a, die für einen Betrieb mit Normaldruck ausgelegt ist, steht, sodass ein Wärmeaustausch des Kopfprodukts der Kolonne 1, die für einen Betrieb mit Überdruck ausgelegt ist, und des Sumpfprodukts der Kolonne 2a, die für einen Betrieb mit Normaldruck ausgelegt ist, ermöglicht wird,
- mindestens einen Wärmetauscher 3b, der in fließender Verbindung mit dem Kopf der Kolonne 2a, die für einen Betrieb mit Normaldruck ausgelegt ist, und mit dem Boden der Kolonne 2b, die für einen Betrieb unter Vakuum ausgelegt ist, steht, sodass ein Wärmeaustausch des Kopfprodukts der Kolonne 2a, die für einen Betrieb mit Normaldruck ausgelegt ist, und des Sumpfprodukts der Kolonne 2b, die für einen Betrieb unter Vakuum ausgelegt ist, ermöglicht wird,
- eine Zuleitung 5 für das Kopfprodukt der Kolonne 1, die für einen Betrieb mit Überdruck ausgelegt ist, mit der der Wärmetauscher 3a mit dem oberen Teil der Kolonne 2a, die für einen Betrieb mit Normaldruck ausgelegt ist, verbunden ist.
Die Zuleitung 5 ist zweckmäßig oberhalb der Zuleitung 7a von einem Teilstrom eines Aceton/Wassergemisches zur Kolonne 2a, die für einen Betrieb mit Normaldruck ausgelegt ist, an der Kolonne 2a angebracht.

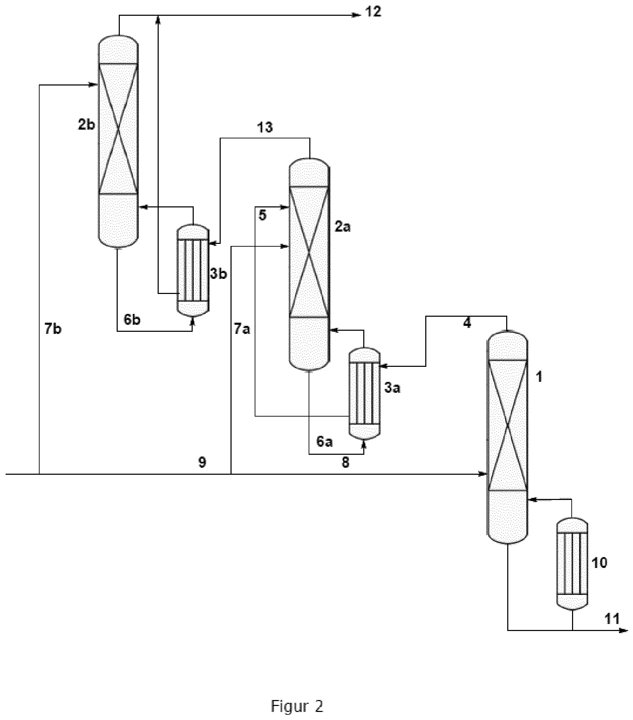

Diese Ausführungsform der erfindungsgemäßen Vorrichtung ist in Fig. 2 gezeigt.

Bei dieser Ausführungsform der Erfindung kann zusätzlich eine weitere Zuleitung vorgesehen sein, mit der das Sumpfprodukt der Kolonne 2a, die mit Normaldruck betrieben wird unterhalb der Zufuhr des Teilstroms des Aceton/Wassergemisches in die Kolonne 2b, die unter Vakuum betrieben wird, eingeleitet werden kann. Dieses zusätzliche Merkmal ermöglicht eine noch stärkere Abreicherung von Aceton im Sumpfprodukt der Kolonne 2a.

### Bezugszeichenliste

- 1: Kolonne für Betrieb bei Überdruck
- 2, 2a, 2b: Kolonne für Betrieb bei Normaldruck
- 3, 3a, 3b: Wärmetauscher
- 4, 4a, 4b: Zuleitung von Kopfprodukt zu Wärmetauscher
- 5: Zuleitung von Kopfprodukt in Kolonne 2
- 6, 6a, 6b: Sumpfproduktkreislauf
- 7, 7a, 7b: Zuleitung für Teilstrom zu Kolonne 2
- 8: Zuleitung für Teilstrom zu Kolonne 1
- 9: Hauptleitung für Aceton/Wassergemisch
- 10: Wärmetauscher
- 11: Ableitung Abwasser
- 12: Ableitung Aceton
- 13: Tank
- 14a, 14b: Zuleitung von Kopfprodukt in Kolonnen 2a und 2b
- 15: Rückführleitung zu Kolonne 1
- 16: Kondensationsvorrichtung
- 17: Kondensationsvorrichtung

## Patentansprüche

1. Verfahren zur Abtrennung von Aceton aus Aceton/Wassergemischen, umfassend
- Aufkonzentrieren eines Teilstroms des Aceton/Wassergemisches in mindestens einer Kolonne, die bei Überdruck betrieben wird, wobei ein Produkt mit einer Acetonkonzentration von mindestens 80 Gew.-% erhalten wird,
- Aufkonzentrieren eines Teilstroms des Aceton/Wassergemisches in mindestens einer Kolonne, die bei Normaldruck betrieben wird,
wobei das Kopfprodukt aus der Kolonne, die bei Überdruck betrieben wird, über einen Wärmetauscher zum Erwärmen des Sumpfprodukts der Kolonne, die unter Normaldruck betrieben wird, genutzt wird, und wobei das Kopfprodukt anschließend oberhalb der Zufuhr des Teilstroms des Aceton/ Wassergemisches in die Kolonne, die unter Normaldruck betrieben wird, eingeleitet wird.

2. Verfahren gemäß Anspruch 1, **dadurch gekennzeichnet, dass** die Kolonne, die bei Überdruck betrieben wird, bei einem Überdruck im Bereich von 4,5 bis 8 bar absolut, bevorzugt 5 bis 7 bar absolut und meist bevorzugt etwa 6 bar absolut betrieben wird.

3. Verfahren gemäß Anspruch 1 oder 2, **dadurch gekennzeichnet, dass** in der Kolonne, die unter Überdruck betrieben wird, ein Kopfprodukt mit einer Acetonkonzentration von mindestens 90 Gew.-%, bevorzugt 90 bis 99,9 Gew.-%, stärker bevorzugt 93 Gew.-% bis 98 Gew.-%, und weiter bevorzugt 94 Gew.-% bis 96 Gew.-% erhalten wird.

4. Verfahren gemäß irgendeinem der vorstehenden Ansprüche, **dadurch gekennzeichnet, dass** das aus der Kolonne, die bei Überdruck betrieben wird, erhaltene Sumpfprodukt zur Vorwärmung des in die Kolonne eingebrachten Aceton/Wassergemisches genutzt wird.

5. Verfahren gemäß irgendeinem der vorstehenden Ansprüche, **dadurch gekennzeichnet, dass** die Kolonne, die bei Überdruck betrieben wird, 30 - 60, bevorzugt 40 - 50 theoretische Böden aufweist.

6. Verfahren gemäß irgendeinem der vorstehenden Ansprüche, **dadurch gekennzeichnet, dass** die Kolonne, die bei Normaldruck betrieben wird, 20 - 40, bevorzugt 25 -35 theoretische Böden aufweist.

7. Verfahren gemäß irgendeinem der vorstehenden Ansprüche, **dadurch gekennzeichnet, dass** das Verfahren des Weiteren umfasst:
- Aufkonzentrieren eines Teilstroms des Aceton/Wassergemisches in mindestens einer Kolonne, die unter Vakuum betrieben wird,
wobei das Kopfprodukt aus der Kolonne, die bei Normaldruck betrieben wird, über einen Wärmetauscher zum Erwärmen des Sumpfproduktes der Kolonne, die unter Vakuum betrieben wird, genutzt wird.

8. Verfahren gemäß Anspruch 7, **dadurch gekennzeichnet, dass** das Sumpfprodukt aus der Kolonne, die bei Normaldruck betrieben wird, unterhalb der Zufuhr des Teilstroms des Aceton/Wassergemisches in die Kolonne, die unter Vakuum betrieben wird, eingeleitet wird.

9. Verfahren gemäß irgendeinem der vorstehenden Ansprüche, **dadurch gekennzeichnet, dass** das Aceton in der mindestens einen Kolonne, die bei Normaldruck betrieben wird, auf eine Konzentration von mindestens 98% Gew.-%, bevorzugt mindestens 98,5 Gew.-%, stärker bevorzugt mindestens 98,5 bis 99,9 Gew.-% aufkonzentriert wird.

10. Verfahren gemäß irgendeinem der vorstehenden Ansprüche, **dadurch gekennzeichnet, dass** die Kolonne, die bei Normaldruck betrieben wird, mit einem Rücklaufverhältnis von 1 bis 2 betrieben wird.

11. Vorrichtung zur Durchführung eines Verfahrens gemäß einem der Ansprüche 1 bis 10, umfassend:
- mindestens eine Kolonne (1) zur Auftrennung eines Teilstroms eines Aceton/Wassergemisches, die für einen Betrieb mit Überdruck ausgelegt ist,
- mindestens eine Kolonne (2) zur Auftrennung eines Teilstroms eines Aceton/Wassergemisches, die für einen Betrieb mit Normaldruck ausgelegt ist,
- Zuleitungen für Teilströme (7,8) eines Aceton/Wassergemisches zu den Trennkolonnen,
- mindestens einen Wärmetauscher (3), der in fließender Verbindung mit dem Kopf der Kolonne (1), die für einen Betrieb mit Überdruck ausgelegt ist, und mit dem Boden der Kolonne (2), die für einen Betrieb mit Normaldruck ausgelegt ist, steht, sodass ein Wärmeaus-tausch des Kopfprodukts der Kolonne (1), die für einen Betrieb mit Überdruck ausgelegt ist, und des Sumpfprodukts der Kolonne (2), die für einen Betrieb mit Normaldruck ausgelegt ist, ermöglicht wird,
- eine Zuleitung (5) für das Kopfprodukt der Kolonne (1), die für einen Betrieb mit Überdruck ausgelegt ist, mit der der Wärmetauscher (3) mit dem oberen Teil der Kolonne (2), die für einen Betrieb mit Normaldruck ausgelegt ist, verbunden ist.

12. Vorrichtung nach Anspruch 11, **dadurch gekennzeichnet, dass** die Zuleitung (5) oberhalb der Zuleitung (7) für einen Teilstrom eines Aceton/Wassergemisches mit der Kolonne, die für einen Betrieb mit Normaldruck ausgelegt ist, verbunden ist.

13. Vorrichtung nach Anspruch 11 oder 12, **dadurch gekennzeichnet, dass** sie eine Kondensationsvorrichtung für in der Kolonne (2), die für einen Betrieb mit Normaldruck ausgelegt ist, generiertes Kopfprodukt aufweist.

14. Vorrichtung nach mindestens einem der Ansprüche 11 bis 13, **dadurch gekennzeichnet, dass** sie einen Wärmetauscher (10) aufweist, der in fließender Verbindung mit dem Sumpfprodukt der Kolonne, die für einen Betrieb mit Überdruck ausgelegt ist, und mit der Zuleitung (8) für den Teilstrom eines Aceton/Wassergemisches zu dieser Kolonne steht.

15. Vorrichtung nach mindestens einem der Ansprüche 11 bis 14, **dadurch gekennzeichnet, dass** die Vorrichtung umfasst:
- mindestens eine Kolonne 1 zur Auftrennung eines Teilstroms eines Aceton/ Wassergemisches, die für einen Betrieb mit Überdruck ausgelegt ist,
- mindestens eine Kolonne 2a zur Auftrennung eines Teilstroms eines Aceton/Wassergemisches, die für einen Betrieb mit Normaldruck ausgelegt ist,
- mindestens eine Kolonne 2b zur Auftrennung eines Teilstroms eines Aceton/Wassergemisches, die für einen Betrieb unter Vakuum ausgelegt ist,
- Zuleitungen für Teilströme 7a, 7b, 8 eines Aceton/Wassergemisches zu den Trennkolonnen,
- mindestens einen Wärmetauscher 3a, der in fließender Verbindung mit dem Kopf der Kolonne 1, die für einen Betrieb mit Überdruck ausgelegt ist, und mit dem Boden der Kolonne 2a, die für einen Betrieb mit Normaldruck ausgelegt ist, steht, sodass ein Wärmeaustausch des Kopfprodukts der Kolonne 1, die für einen Betrieb mit Überdruck ausgelegt ist, und des Sumpfprodukts der Kolonne 2a, die für einen Betrieb mit Normaldruck ausgelegt ist, ermöglicht wird,
- mindestens einen Wärmetauscher 3b, der in fließender Verbindung mit dem Kopf der Kolonne 2a, die für einen Betrieb mit Normaldruck ausgelegt ist, und mit dem Boden der Kolonne 2b, die für einen Betrieb unter Vakuum ausgelegt ist, steht, sodass ein Wärmeaustausch des Kopfprodukts der Kolonne 2a, die für einen Betrieb mit Normaldruck ausgelegt ist, und des Sumpfprodukts der Kolonne 2b, die für einen Betrieb unter Vakuum ausgelegt ist, ermöglicht wird,
- eine Zuleitung 5 für das Kopfprodukt der Kolonne 1, die für einen Betrieb mit Überdruck ausgelegt ist, mit der der Wärmetauscher 3a mit dem oberen Teil der Kolonne 2a, die für einen Betrieb mit Normaldruck ausgelegt ist, verbunden ist.
